# EUROPEAN PATENT APPLICATION

(11) **EP 1 647 547 A1**
(43) Date of publication of application: **19.04.2006**
(21) Application number: 05468015.2
(22) Date of filing: 13.10.2005
(51) Int. Cl.: C07D 209/42, A61K 31/404, A61P 9/00

(54) **New hydrated crystalline forms of perindopril erbumine, process for the preparation thereof and pharmaceutical formulations containing these compounds**

(30) Priority: 15.10.2004 SI 200400285
(71) Applicant: Diagen Smartno pri Ljubljani, d.o.o., 1211 Ljubljana Smartno (SI)
(72) Inventor: Rucman, Rudolf, 1211 Ljubljana Smartno (SI); Zupet, Pavel, 8000 Novo mesto (SI)
(74) Representative: Primozic, Alenka

(57) **Abstract**

The object of the invention are new crystalline forms perindopril erbumine monohydrate, perindopril erbumine sesquihydrate and perindopril erbumine dihydrate and a process for the preparation thereof by dissolving perindopril erbumine in water or in water under the addition of a volatile water-miscible polar organic solvent, freezing and lyophilizing.

Another object of the invention is a new process for the preparation of perindopril erbumine monohydrate in pure crystalline form by freezing aqueous acetone solutions and lyophilizing.

Another object of the invention are pharmaceutical formulations for the treatment of arterial hypertension and with vasodilatory activity, containing a therapeutically effective amount of these new crystalline forms.

## Description

The invention belongs to the field of organic chemistry synthesis and relates to new hydrated crystalline forms of perindopril erbumine as well as to the preparation thereof. Because of significant pharmaceutical importance of perindopril erbumine there was a need for an industrially applicable process for preparing it in new stable crystalline forms suitable for the manufacture of pharmaceutical preparations.

The object of the invention are new hydrated forms of perindopril erbumine of the general formula I 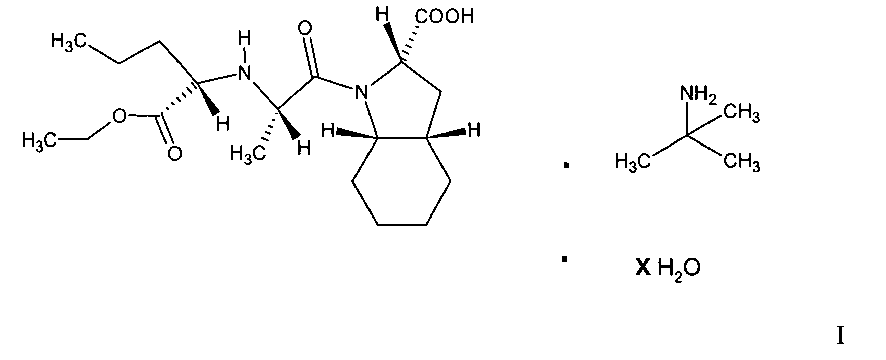 wherein X means:
1.0 for monohydrate,
1.5 for sesquihydrate, and
2.0 for dihydrate.

### Prior Art

Perindopril erbumine of the formula II 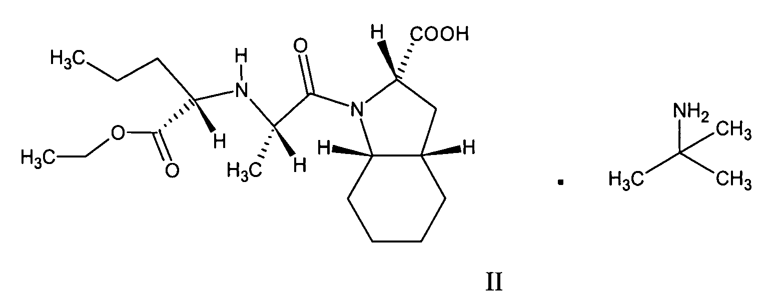 is 2-methylpropane-2-amine-(2S,3aS,7aS)-1-[(2S)-2-[[(1S)-1-ethoxycarbonyl)-butyl]amino]propanoyl]octahydro-1*H*-indole-2-carboxylate.

Perindopril erbumine is an important ACE inhibitor, which is used as a very succesful drug for the treatment of hypertension.

Perindopril was first synthesised in 1981 and disclosed in EP 0049658 B1 and US 4,508,729. The synthesis is complicated and a stereoisomer mixture is formed, which must be separated. The coupling reagent for the peptide bond is DCC, N,N'-dicyclohexylcarbodiimide. Also the substance as a pure (S) isomer and its sodium salt are claimed therein.

In EP 0308341 B1 (1987) a process for the preparation of perindopril-t-butylamine salt by reacting N-[(S)-carbethoxy-1-butyl]-(S)-alanine with (2S,3aS,7aS)-octahydroindole-2-carboxylic acid benzyl ester is claimed. The condensation reagent is DCC, N,N'-dicyclohexylcarbodiimide. The protecting benzyl group is eliminated by catalytic hydrogenation. The process is critical because of high risk of epimerization. In both processes mentioned there are difficulties with eliminating N,N'-dicyclohexylurea.

An industrial stereoselective synthesis is disclosed in US 4,902,817.

A synthesis of (2S,3aS,7aS)-octahydroindole-2-carboxylic acid by hydrogenation of (S)-2-carbethoxyindoline is disclosed in US 4,914,214 (1988). In the synthesis of perindopril, N-[(S)-carbethoxy-1-butyl]-(S)-alanine is activated with DCC, N,N'-dicyclohexylcarbodiimide, in the presence of 1-hydroxybenzotriazole, or with N,N'-carbonyldiimidazole. A disadvantage of the process is the formation of the side product N,N'-dicyclohexylurea, which has to be eliminated from the reaction mixture together with 1-hydroxybenzotriazole.

In EP 1279665 A2 (2001) there is claimed a process of synthesis of perindopril erbumine via an intermediate formation of oxazolidine obtained by the reaction of N-[(S)-carbethoxy-1-butyl]-(S)-alanine with phosgene or triphosgene. This intermediate then reacts with (2S,3aS,7aS)-octahydroindole-2-carboxylic acid to form perindopril. It is further dissolved in an organic solvent and, under addition of erbumine, converted to the perindopril erbumine. A disadvantage of this synthesis is the use of the poisonous phosgene.

In SI 9500140 (1995) there is disclosed a synthesis of perindopril starting from N-[(S)-carbethoxy-1-butyl]-(S)-alanine with an intermediate formation of an N-sulfoxy derivative, which further reacts with a silylated form of (2S,3aS,7aS)-octahydroindole-2-carboxylic acid to perindopril. The formation of chlorothionylimidazole is problematic since more than one compound is formed. The further course of the reaction is problematic as well because this reagent is sensitive to moisture and because the reaction may take place via imidazolide.

In WO 03/064388 (2003) there is disclosed a synthesis of perindopril by an intermediate formation of N-[(S)-carbethoxy-1-butyl]-(S)-alanine acid chloride with a protected NH group and a subsequent reaction with (2S,3aS,7aS)-octahydroindole-2-carboxylic acid. The acid chloride is formed by using thionylchloride at ambient or slightly elevated temperature. The NH group is protected with an N-alkoxycarbonyl group e.g. N-ethoxycarbonyl, N-methoxycarbonyl, N-benzyloxycarbonyl group. The deficiencies of this synthesis are, on the one hand, a low stability of N-alkoxycarbonyl groups in conditions of strong acidic chlorination (these groups disintegrate already here) and, on the other hand, a too great stability of the N-benzyloxycarbonyl group, which needs to be eliminated by catalytic hydrogenolysis. The catalytic hydrogenolysis is not mentioned in the synthesis and therefore the given course of the synthesis is not likely to be possible.

There are known several other processes for the synthesis of perindopril, which differ from each other in their economicalness and product quality.

### Technical Problem

In recent years several crystalline forms of perindopril erbumine with their typical X-ray powder diffractograms have been successfully prepared and patents applied for.

In WO 01/87835 A1 (2001) to Servier, a new α-crystalline form of perindopril erbumine as well as a process for the preparation thereof by crystallization from ethyl acetate are disclosed.

In WO 01/87836 A1 (2001) to Servier, a new β-crystalline form and its preparation by crystallization from dichloromethane are disclosed.

In WO 01/83439A2 (2000) to Servier, a new γ-crystalline form and its preparation by crystallization of perindopril erbumine from chloroform are disclosed.

Pharmaceutical forms prepared from these crystalline structures of perindopril erbumine are disclosed as well.

The company AZAD Fine Chemicals AG has announced the discovery and preparation of new δ- and ε-forms of perindopril erbumine.

All disclosed new polymorphic forms of perindopril erbumine exclusively relate to crystallized perindopril erbumine in non-solvated form.

Recently a new form of perindopril erbumine as monohydrate has been disclosed in GB 2,395,195, wherein the preparation thereof by crystallization from organic solvents with addition of a small amount of water is disclosed as well.

### Solution to the Technical Problem

There have been studied several possibilities to provide new crystalline structures of perindopril erbumine with improved physico-chemical properties, especially its stability, and thus to facilitate the preparation of a pharmaceutical form.

The object of the invention are three novel crystalline forms of the formula I, especially:
perindopril erbumine sesquihydrate of the formula III 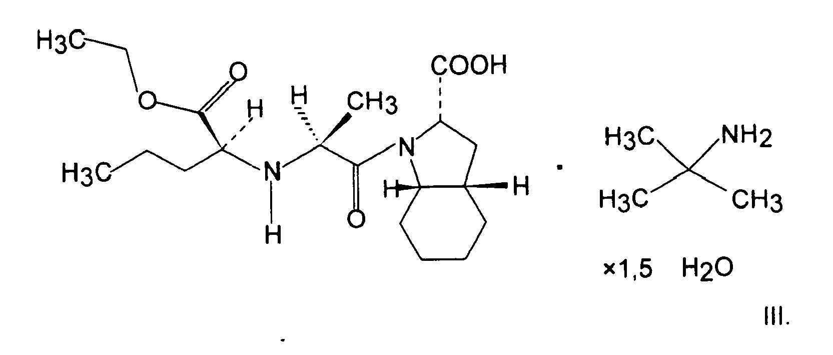
and perindopril erbumine dihydrate of the formula IV

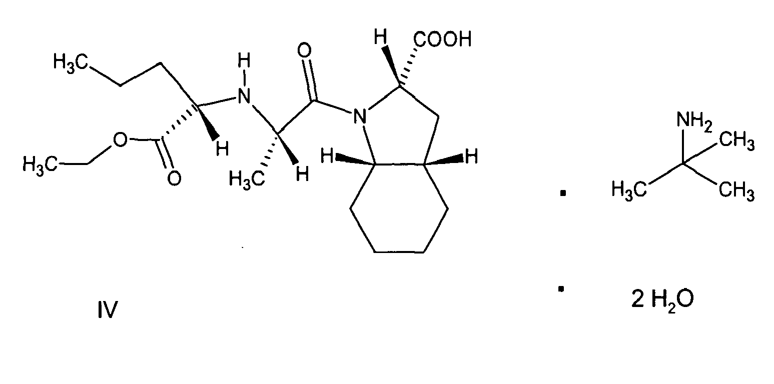

Perindopril erbumine sesquihydrate of the formula III is prepared in such a way that an aqueous solution of perindopril erbumine is slowly cooled and frozen and then the solvent is removed by lyophilization. The obtained lyophilizate is left to stand in humid air for a few hours to achieve the balance of water content and the partly amorphic residue is crystallized.

Perindopril erbumine dihydrate of the formula IV is prepared in a similar manner with the only difference that up to 20% of a volatile water-miscible organic solvent are added. The solution is cooled to freeze and the frozen solvents are removed by lyophilization.

The new crystalline forms are extremely well soluble in water, their solubility is practically unlimited.

Perindopril erbumine dihydrate is also prepared by slow crystallization from a saturated solution of perindopril erbumine in water.

One object of the present invention is also a new process for the preparation of perindopril erbumine monohydrate of the formula I (X = 1.0). According to this new process perindopril erbumine monohydrate can be prepared by lyophilization of perindopril erbumine solutions in a solvent mixture containing water and acetone or isopropanol. This process is remarkably better than previously known methods because of its significantly higher yield (about 100%) - there is no loss of the substance in crystallization mother liquors.

For the preparation of hydrates according to the invention, aqueous solutions or water/solvent solutions of perindopril erbumine in concentrations from 2-10%, more favourable 5%, most favourable 10% are used.

As the starting material perindopril erbumine may be used in any form, it can be also amorphous, or perindopril erbumine is prepared in situ from perindopril and tert-butylamine.

The yields of the process are very high, ranging from 95% to 100%.

The new hydrates of perindopril erbumine were investigated by X-ray powder diffraction method on Siemens D500 instrument with reflection method at set parameters: CuKα emission, angle range 2θ from 2° to 37°, step 0.04°, integration time 1 sec.

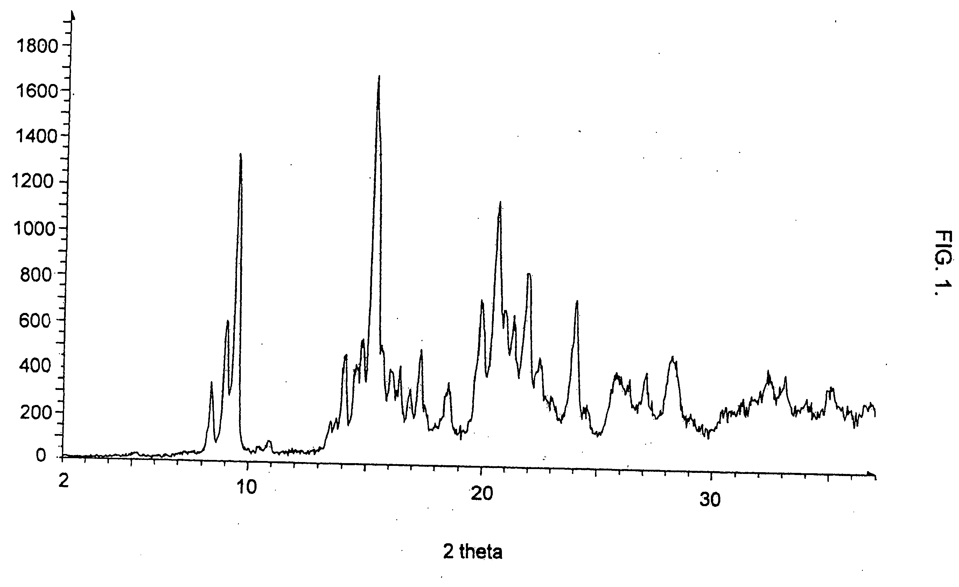

X-ray diffractogram of perindopril erbumine sesquihydrate, Fig. 1, exhibits typical diffraction signals at the following angles 2θ: 8.976; 9.425; 14.821; 15.253; 19.924; 20.582; 20.960; 21.324 and 21.933 (only main signals with an intensity of more than 40% are listed).

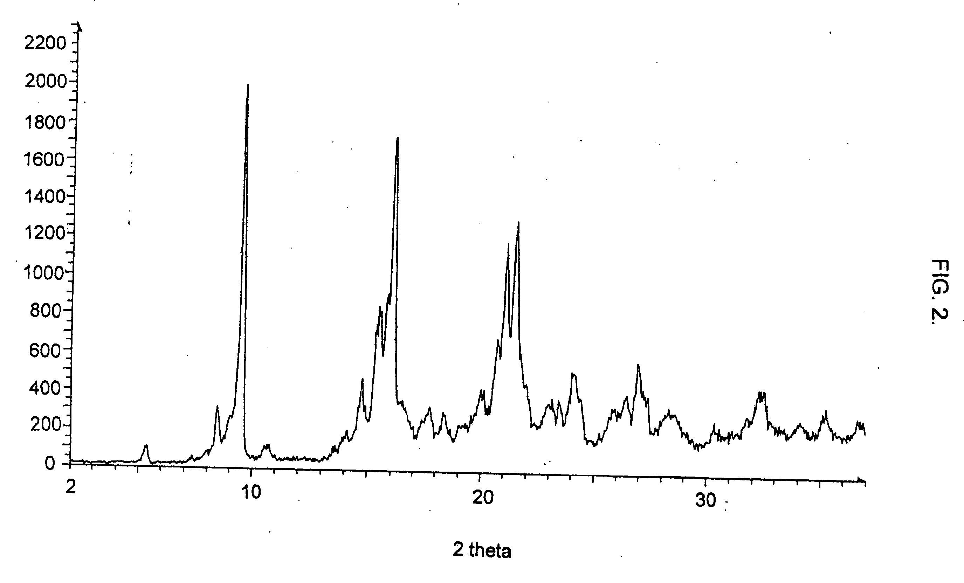

X-ray diffractogram of perindopril erbumine dihydrate, Fig. 2, exhibits typical diffraction signals at the following angles 2θ: 9.470; 15.488; 15.760; 16.050; 21.036 and 21.460 (main signals with an intensity of more than 40%).

The shown diffractograms, Figs. 1 and 2, essentially differ from the diffractograms of all known crystalline forms of perindopril erbumine and also of the known monohydrate form.

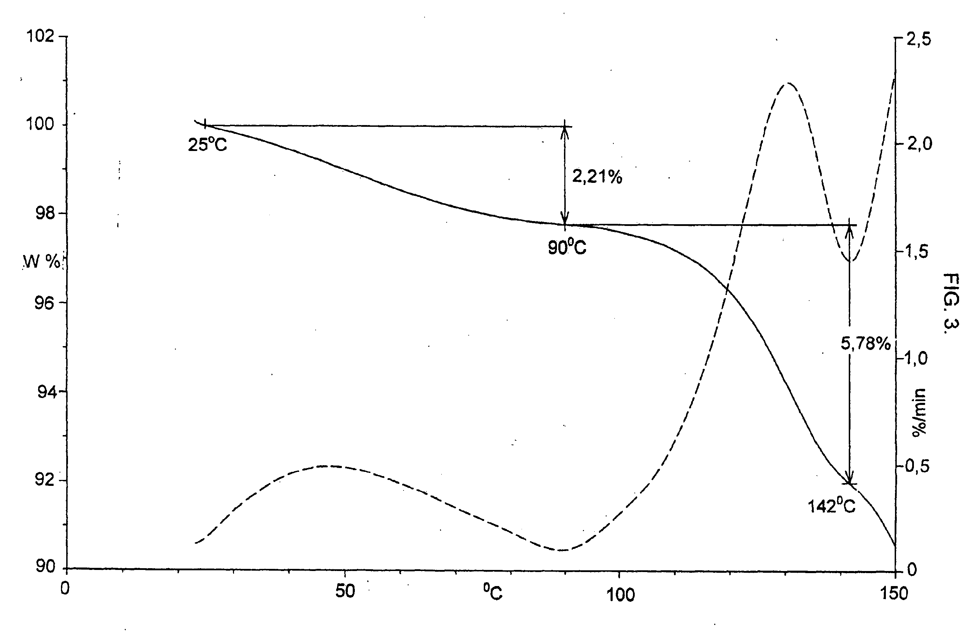

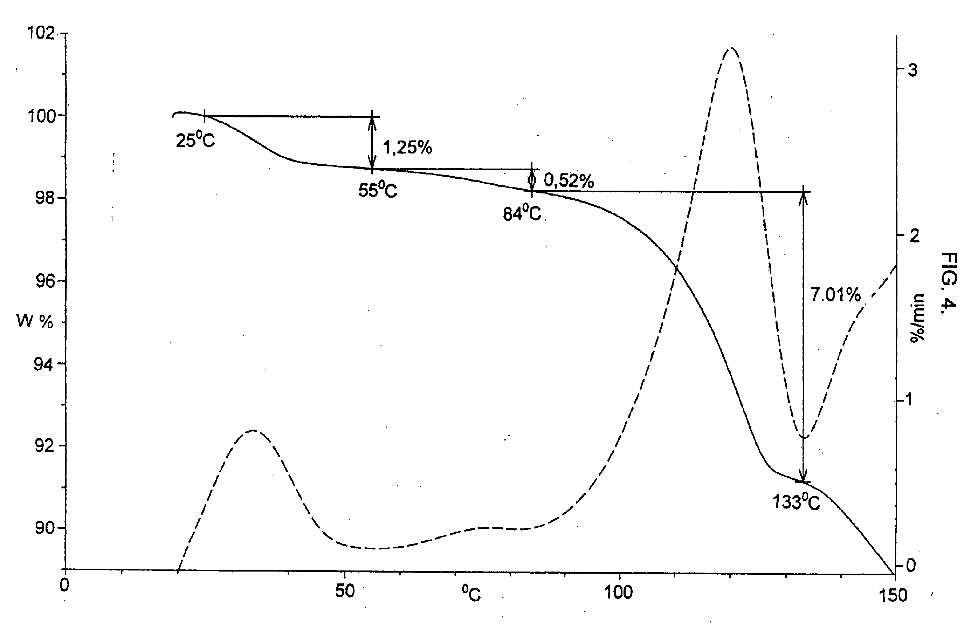

The thermical analyses DSC and TGA were performed on an instrument of TA Instruments in the range from 20°-150°C (Fig. 3 and 4).

Perindopril erbumine sesquihydrate, Fig. 3, shows a loss of 5.78% of water in the range of 90° to 142°C and thus the ratio of 1.5 mol of water to 1 mol of the compound is confirmed.

Perindopril erbumine dihydrate, Fig. 4, shows a loss of 7.01 % of water in the range of 84° to 133°C and of 7.53% in the broader range from 55° to 133°C, which indicates the ratio of 2 mol of water to 1 mol of the compound.

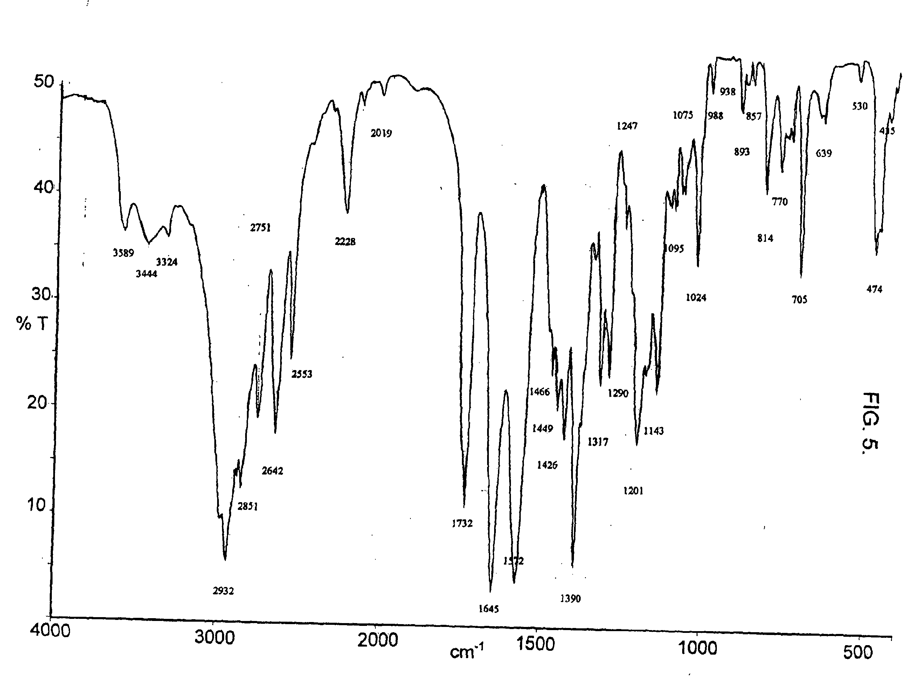

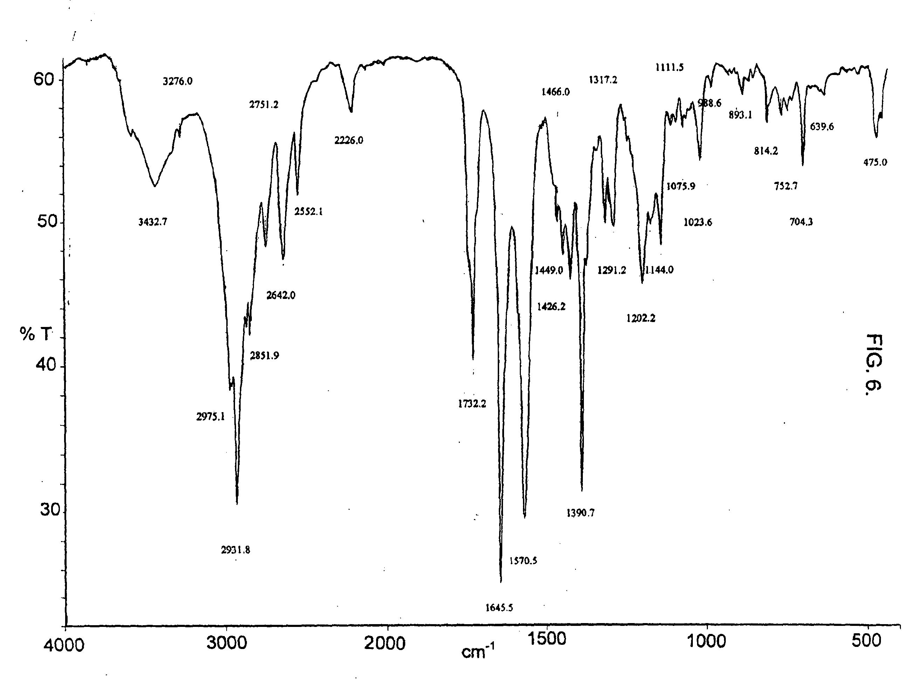

Infrared spectra recorded with KBr technique, Fig. 5 for perindopril erbumine sesquihydrate and Fig. 6 for perindopril erbumine dihydrate, also indicate a higher water content than for perindopril erbumine alone.

The invention also relates to pharmaceutical formulations with antihypertensive and vasodilatory activity, which contain a therapeutically effective amount of perindopril erbumine monohydrate, sesquihydrate or dihydrate. Suitable pharmaceutical formulations are especially solid dosage forms such as tablets with immediate or delayed release of the active drug, dispersable tablets, orodispersable tablets or capsules.

The present invention is illustrated by the following Examples, but not limited thereto.

### Example 1

Perindopril erbumine (6.0 g; 13.6 mmol) was dissolved in water (60 ml) and filtered. The solution was cooled to -15°C until it was frozen and left to stand at this temperature for 1 hour. It was lyophilized *in vacuo* at 0.1 to 1 mbar. The white residue was left to stand in the air with a relative humidity of more than 75% for another 2 hours. A white substance with a melting range of 132° to 138°C (Kofler) was obtained, which contained 1.5 mol water to 1 mol of the substance. The X-ray diffractogram and DSC/TGA thermogram corresponded to perindopril erbumine sesquihydrate.

### Example 2

Perindopril erbumine (0.5 g; 1.13 mmol) was dissolved in water (4 ml) and acetonitrile (1 ml) and filtered. The solution was cooled in a methanol cooling bath to -45 to -50°C unil it was frozen. It was lyophilized *in vacuo* in the range of 0.1 to 1 mbar. The theoretical amount of a white substance with m.p. of 121° to 126°C was obtained. It was confirmed by X-ray diffractogram and DSC/TGA thermogram that the substance was perindopril erbumine dihydrate.

### Example 3

Perindopril (1.0 g, 2.26 mmol) was dissolved in isopropanol (1 ml) and water (9 ml) containing tert-butylamine (0.24 ml; 2.3 mmol) was added under stirring. The mixture was stirred to a complete dissolution and the solution was filtered. The solution was then frozen at -45°C. It was lyophilized in the same way as in Example 1. A white substance with the same characteristics as stated in Example 1 was obtained.

### Example 4

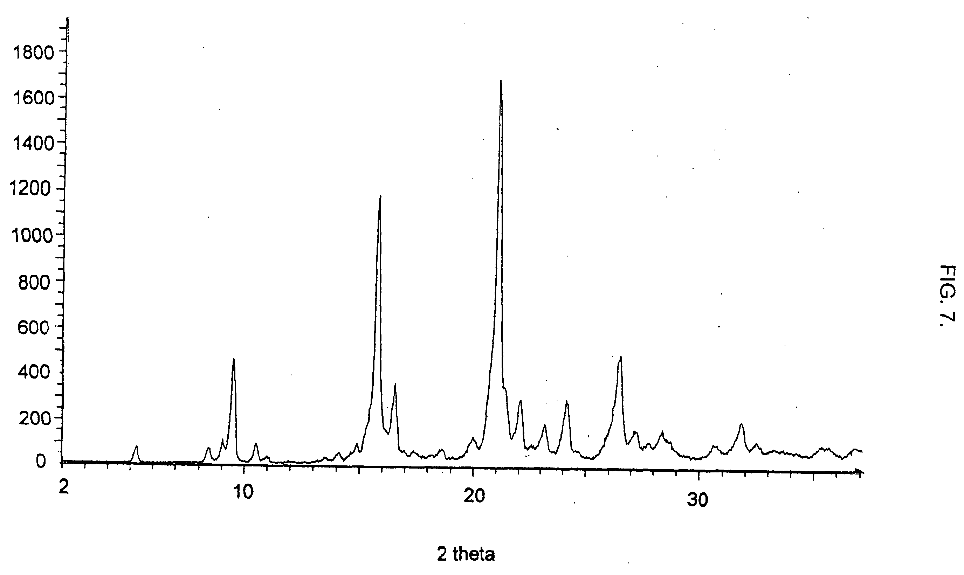

Perindopril erbumine (10 g; 22.6 mmol) was dissolved in a solvent mixture (100 ml) containing water (60 ml) and acetone (40 ml) and then slowly cooled down to -80°C until crystallization and solidification occurred. Then the solid frozen mass was dried by lyophilization *in vacuo.* There was obtained perindopril erbumine monohydrate (10.4 g, 100%), which contained 3.95% water and showed an X-ray diffractogram as given in Fig. 7.

### Example 5

Perindopril erbumine (1 g; 2.26 mmol) was dissolved in water (5 ml) and left to stand in the air at a temperature between 30° and 40°C and a relative humidity between 50% and 70% for at least 10 days unil the solid crystalline substance perindopril erbumine dihydrate (1.1 g; 100%) containing 8.4% of water was obtained.

### Example 6

Composition of a tablet with 5 mg of the active drug:

| | |
|---|---|
| perindopril erbumine sesquihydrate | 5 mg |
| lactose | 30 mg |
| maize starch | 15 mg |
| microcrystalline cellulose | 49 mg |
| magnesium stearate | 1 mg |
| total mass of the tablet | 100 mg |

The tablets were prepared by blending the ingredients, wetting, granulating, drying and compressing the mixture into tablets in an appropriate device.

## Claims

1. New crystalline perindopril erbumine hydrated forms of the formula 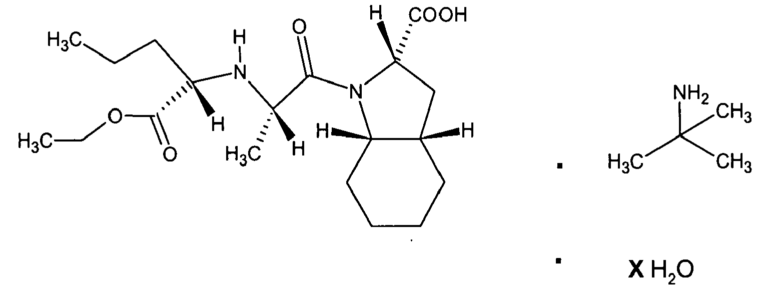 wherein X means 1.0, 1.5 or 2.0.

2. Perindopril erbumine sesquihydrate according to claim 1 with 1.5 mol of water to 1 mol of the substance, **characterized by** main diffraction signals in X-ray powder diffraction spectrum at the following angles 2θ: 8.976; 9.425; 14.821; 15.253; 19.924; 20.582; 20.960; 21.324 and 21.933.

3. Perindopril erbumine dihydrate according to claim 1 with 2 mol of water to 1 mol of the substance, **characterized by** main diffraction signals in X-ray powder diffraction spectrum at the following angles 2θ: 9.470; 15.488; 15.760; 16.050; 21.036 and 21.460.

4. Process for the preparation of perindopril erbumine sesquihydrate according to claims 1 and 2, **characterized in that** an aqueous solution of perindopril erbumine with a concentration of from 2 to 20% is frozen and dried by lyophilization *in vacuo*.

5. Process for the preparation of perindopril erbumine dihydrate according to claims 1 and 3, **characterized in that** an aqueous solution of perindopril erbumine containing also up to 20% of a volatile water-miscible polar organic solvent is frozen and dried by lyophilization *in vacuo*.

6. Process according to claim 5, **characterized in that** a lower aliphatic alcohol with 1 to 4 carbon atoms, acetonitrile, acetone or tetrahydrofuran, preferably acetonitrile, are used as the volatile polar organic solvent.

7. Process for the preparation of perindopril erbumine dihydrate according to claims 1 and 3, **characterized by** a slow crystallization of the substance from a saturated aqueous solution in the air.

8. Process for the preparation of a pure perindopril erbumine monohydrate according to claim 1, **characterized in that** perindopril erbumine is dissolved in a solvent mixture containing water and acetone in ratio of from 30 : 70 to 70 : 30, the solution is then slowly cooled to -80°C to solidify and finally dried by lyophilization.

9. Pharmaceutical formulations with antihypertensive and vasodilatory activity, **characterized in that** they contain a therapeutically effective amount of perindopril erbumine monohydrate, sesquihydrate or dihydrate according to claim 1 together with pharmaceutically acceptable carriers and other auxiliary substances.
